# EUROPEAN PATENT APPLICATION

(11) **EP 4 671 315 A2**
(43) Date of publication of application: **31.12.2025**
(21) Application number: 25215454.7
(22) Date of filing: 16.02.2023
(51) Int. Cl.: C08J 5/08

(54) **BIOCOMPATIBLE AND RESORBABLE COMPOSITE MATERIAL AND METHOD FOR OBTAINING SUCH**

(30) Priority: 16.02.2022 US 202263310850 P; 25.02.2022 EP 22158902
(62) Divisional of application: 23705010.9
(71) Applicant: Purac Biochem B.V., 4206 AC Gorinchem (NL)
(72) Inventor: HASAN, Muhammed Sami, TUCKER, GA, 30084 (US); CANADELL-AYATS, Judit, 4206 AC GORINCHEM (NL); LIU, Jenq, TUCKER, GA, 30084 (US); DE VOS, Siebren Cornelis, 4206 AC GORINCHEM (NL); PENISTON, Shawn James, TUCKER, GA, 30084 (US)
(74) Representative: De Vries & Metman

(57) **Abstract**

The invention pertains to a composite material, comprising a plurality of compatible glass fibers in a thermoplastic polymer matrix, wherein the glass fibers and the polymer matrix are biocompatible and resorbable. The invention further pertains to a method for obtaining a solid polymer composite material comprising a plurality of biocompatible and resorbable glass fibers ,which are embedded in a biocompatible and resorbable matrix polymer, wherein the matrix polymer is applied from solution and the solvent of said solution is at least partially removed using an anti-solvent.

## Description

### Field of the invention

The invention relates to a method for obtaining a solid polymer composite material comprising a plurality of biocompatible and resorbable glass fibers which are embedded in a biocompatible and resorbable matrix polymer. The invention further relates to a glass fiber reinforced polymer composite obtained by said method and a medical device comprising said composite material.

### Background

The field of medical devices such as implants is a very active and fast-growing field. As the average human age continues to rise, the field is expected to continue to continue to grow in the coming years. As a result, there is a significant push for improving the properties of medical implants and for improving the methods for manufacturing medical implants.

Many types of material are known for their use for producing medical implants, such as metal alloys, ceramics, and polymers. Metal alloys are the traditional choice for making orthopedic implants such as pins, screws, and plates as these are very well suited to carry an external load owing to their strength. However, metal alloys are typically stiffer (i.e., they have a higher modulus of elasticity) than the bone to which they are connected. As a result, a decrease in bone density at the repair site occurs due to stress-shielding. Also, metal alloys typically do not degrade inside the body. Hence to avoid bone resorption and due to their lack of resorption, metal alloys generally need to be removed after bone healing, which requires a second surgery.

New materials have been developed to better match the mechanical properties of the bone and which can be resorbed by the body over time. These materials minimize the stress-shielding problem and eliminate the need for a second surgery.

EP2243500 describes how to manufacture an implantable composite material comprising a polymer matrix and glass fibers. Here the glass fibers are separately produced and then chopped to 10 mm length before being mixed with the matrix polymer in an extruder. Alternatively, the fibers were separately produced and mixed with molten polymer in a cross-head die. In either case, the matrix polymer must be in the molten state in order to be processed in the extruder, meaning that the matrix material needs to be a thermoplastic resin which needs to be brought to a relatively low viscosity using high processing temperatures. The process of EP2243500 is hence limited as the matrix polymer needs to be a thermoplastic polymer. In addition, the heat required to achieve the required low viscosity of the thermoplastic polymer can cause thermo-oxidative damage. As a result, the matrix polymer will suffer from polymer degradation and an associated decrease in molecular weight, which translates in reduced mechanical performance and shorter in vivo resorption time.

There is thus a need for a better method of producing polymer-based composite materials that allows for better control over the composition and performance of the composite end product.

### Summary of the invention

The above-described challenges are solved using the method of the invention. This method, for obtaining a solid polymer composite comprising a plurality of biocompatible and resorbable glass fibers which are embedded in a biocompatible and resorbable matrix polymer, comprises:
a) providing a plurality of the glass fibers compatible with the matrix polymer,
b) providing a mixture comprising the matrix polymer in a solvent,
c) applying the mixture onto the plurality of glass fibers, and
d) removing the solvent from the mixture using an anti-solvent to obtain the solid composite material.

As will be understood, the above method eliminates the need for heat-driven, melt-processing and for using an extruder to supply a polymer melt to a melt pultrusion die. Hence there is no longer a need for heating the matrix polymer to a high temperature, which significantly reduces the risk of polymer degradation. Instead, the matrix polymer solution can be applied at a relatively low and easy to handle temperature, such as room or ambient temperature, where polymer degradation is not initiated. In addition, the glass fibers can be oriented and spread as needed and an easy-to-handle mixture comprising a matrix polymer and a solvent can be applied. If needed, multiple mixtures can be applied leading to a thicker layer of matrix polymer.

It is possible to use the method according to the invention with discontinuous (e.g. chopped) fibers. In addition and/or alternatively, the above method may also be used with longer (e.g. continuous or woven or knitted) fibers, which may be beneficial as this allows to better tailor the properties of the glass reinforced polymer composite (GRP) material, and hence does not require breaking the glass fibers in such relatively short fibers.

The invention further relates to a polymer composite material comprising a plurality of biocompatible and resorbable glass fibers in a biocompatible and resorbable polymer matrix, wherein the composite has a matrix polymer monomer content lower than 2 wt.%, more preferably 1 wt.% and even more preferably 0.5 wt.%, with respect to the matrix polymer. A composite with a low monomer content is beneficial as a high monomer content is an indication of undesirable degradation of matrix polymer which can lead to loss of mechanical strength in an uncontrolled manner of products, such as medical implants, produced from the polymer composite material. The composite can be obtained by or obtainable by the method of the invention.

The composite of the invention is particularly useful in medical devices, such as medical implants, for example orthopedic implants and scaffolds.

### Detailed description

The invention relates to a method for obtaining a solid polymer composite material comprising a plurality of biocompatible and resorbable glass fibers which are embedded in a biocompatible and resorbable polymer matrix, comprising the steps of:
a) providing a plurality of the glass fibers compatible with the matrix polymer,
b) providing a mixture comprising the matrix polymer in a solvent,
c) applying the mixture onto the plurality of glass fibers, and
d) removing the solvent from the mixture that has been applied onto the plurality of glass fibers using an anti-solvent to obtain the solid polymer composite material.

It has been found that the method of the present invention has a number of advantages over the use of conventional methods in which the polymer is used in the melt. A first advantage is that it is not necessary to process the matrix polymer at melt temperatures. This is attractive because it decreases the chances of thermal degradation of the polymer. A further advantage is that the use of matrix polymer in solvent may make for a low-viscosity solution, with improved fiber spreading and fiber wetting properties. A further advantage of the use of a solvent-antisolvent combination is that the matrix polymer as it is present in a final product may have a lower content of monomers and low molecular weight oligomers than the starting matrix polymer. As it is the high molecular weight polymer which is responsible for the properties of the composite, a decreased amount of low molecular weight components is considered advantageous.

Biocompatible and resorbable glass fibers are known in the art. The term resorbable in this context means that the material disappears from the body through mineralization (i.e. breakdown and ionic release) and dissolution of the ionic products whilst not significantly activating an inflammatory response during its decomposition process.

Within the context of the present specification, the term composite, also sometimes polymer composite, refers to a composite material comprising glass fiber embedded in a polymer matrix.

The terms used in this application, if not otherwise defined, are those agreed on at the consensus conference on biomaterials in 1987 and 1992, see Williams, DF (ed.): Definitions in biomaterials Proceedings of a consensus conference of the European Society for Biomaterials, Chester, England. March 3-5, 1986. Elsevier, Amsterdam 1987 , and Williams DF, Black J, Doherty PJ. Second consensus conference on definitions in biomaterials. In: Doherty PJ, Williams RL, Williams DF, Lee AJ (eds). Biomaterial-Tissue Interfaces. Amsterdam: Elsevier, 1992 .

In the context of the current invention, compatibility of the glass fibers with the matrix polymer relates to the interface between the glass fibers and the surrounding matrix polymer. It is thus understood that the glass fibers are preferably compatible with the matrix polymer. Therefore, it is preferred that the surface of the glass fibers, which are essentially hydrophilic, is made compatible with the matrix polymer that is in contact with the surface of the glass, which is essentially hydrophobic. Typically, the glass fibers are covered by a coating or sizing layer to lower surface energy. For the concept of compatibility, this coating or sizing layer is considered an integral part of the glass fiber. As a result, a coated or sized glass fiber that is compatible with the matrix polymer layer means that the coating or sizing layer is compatible with the matrix polymer, and thus provides good interfacial adhesion for load-transfer. Hence, in a preferred embodiment the resorbable glass fibers compatible with the matrix polymer are resorbable glass fibers, having a coating or sizing layer on their surface that is compatible with the matrix polymer.

In the method of the invention, a mixture is provided comprising the matrix polymer in a solvent. In the present invention, a solution is generally used, i.e., the polymer is fully dissolved in the solvent. The solvent is a fluid in which the matrix polymer dissolves in concentrations that lead to pourable solutions with dynamic viscosities which allow easy processing. The use of a solution makes it possible to have a processable liquid without having to resort to the higher temperatures necessary for melting the polymer. The solutions can be made using any known method and equipment, such as providing a vessel comprising the matrix polymer and the solvent and mixing the matrix polymer and the solvent using a suitable polymer mixer. It is noted that the mixture may contain solid components, e.g., hydroxy-apatite or calcium phosphate.

It is preferred that the dynamic viscosity of the mixture as it is applied onto the plurality of glass fibers, when measured at 18°C, is preferably at most 10000, more preferably at most 5000 and most preferably at most 500 pascal-seconds (Pa.s), where 1 Pa.s equals 1000 centipoise (cP). The dynamic viscosity of the solution, when measured at 18°C, is preferably in the range of 1 - 1000, preferably 5 - 500, more preferably 20 - 200 Pa.s. It is hereby understood that the viscosity is measured according to ASTM D2196-20, for example using an Anton-Paar ViscoQC Rotational Viscometer.

The required viscosity of the mixture at the time point it is contacted with the plurality of glass fibers can be ensured through the control of various parameters, int.al., the nature of the solvent, the concentration of the polymer, and the age of the solution. It is well known that polymer solutions, especially polymer solutions with higher concentrations, may age over time, resulting in polymer alignment which may lead to an increase in viscosity. If this is the case, the polymer aggregation may be reduced, with an associated reduction of viscosity, by agitation of the mixture e.g., through stirring, shaking, or vibration, optionally with some slight increase in temperature.

The concentration of matrix polymer in the mixture is typically at least 1 wt.%, preferably at least 5 wt.%, more preferably at least 10 wt.% and, even more preferably at least 20 wt.%. The concentration of matrix polymer in the mixture is typically at most 70 wt.%, preferably at most 50 wt.%, more preferably at most 35 wt.% and even more preferably at most 25 wt.%.

In the context of the invention, the term anti-solvent is used to indicate a fluid or liquid which is miscible with the solvent, but in which the matrix polymer, is not soluble under the conditions prevailing during the presence of the anti-solvent. In other words, the anti-solvent that is used for solidification of the matrix polymer is miscible with the solvent, but the matrix polymer is not soluble in that anti-solvent. Because the solvent and the anti-solvent are miscible, the addition of anti-solvent can be used to decrease the solubility of the matrix polymer in the liquid.

In the method of the present invention, the addition of the anti-solvent ensures that the matrix polymer solidifies. This solidification process of the invention may also be described in terms of solvent quality, where the term solvent quality is used in its conventional meaning and indicates how good a certain compound dissolves in a solvent. The solvent quality of the solvent for the matrix polymer is good, meaning that a large amount of matrix polymer can be dissolved in the solvent. On the other hand, the solvent quality of the anti-solvent for the matrix polymer is bad, meaning that the amount of polymer that will dissolve in the anti-solvent is low. When the anti-solvent is added to the mixture of matrix polymer and solvent on the plurality of glass fiber, the solvent quality of the solvent on the glass fibers is lowered. Consequently, by adding the anti-solvent, the solvent quality of the solvent mixture for the matrix polymer deteriorates causing the polymer to gel and solidify by phase separation from the solvent mixture. As a result, the matrix polymer will precipitate onto the glass fibers resulting in a solid polymer composite material.

It is understood that the preferred choice of solvent depends on the matrix polymer used and the preferred anti-solvent (mixture) depends on the choice of solvent and matrix polymer. Matrix polymers are known in the art and any known matrix polymer can be used for the method according to the invention as long as it is suitable for the invention, i.e. it is at least biocompatible and resorbable as well as compatible with the glass fibers.

Preferably the matrix polymer is a polyester and more preferably the matrix polymer is a thermoplastic polyester. Most preferably the matrix polymer is a polyester based on lactide. Additionally or alternatively the matrix polymer is selected from the group consisting of polylactides (PLA), poly-L-lactide (PLLA), poly-DL-lactide (PDLLA), polyglycolide (PGA), poly(ε-caprolactone) (PCL), copolymers of glycolide, glycolide/trimethylene carbonate copolymers (PGA/TMC), lactide/tetramethylglycolide copolymers, lactide/trimethylene carbonate copolymers, lactide/d-valerolactone copolymers, lactide/ε-caprolactone copolymers, L-lactide/DL-lactide copolymers (PLDLA), glycolide/L-lactide copolymers (PLGA), polylactide-co-glycolide, lactide/glycolide/trimethylene carbonate terpolymers, lactide/glycolide/ε-caprolactone terpolymers, PLA/polyethylene oxide copolymers, unsymmetrically 3,6-substituted poly-1,4-dioxane-2,5-diones, polyhydroxybutyrates (PHB), PHB/β-hydroxyvalerate copolymers (PHB/PHV), poly-β-hydroxypropionate (PHPA), poly-p-dioxanone (PDO), poly-d-valerolactone-poly-ε-caprolactone, poly(ε-caprolactone-DL-lactide) copolymers, methyl methacrylate-N-vinyl pyrrolidone copolymers, polyester amides, polyesters of oxalic acid, polydihydropyrans, polyalkyl-2-cyanoacrylates, polyurethanes (PU), polyvinyl alcohol (PVA), polypeptides, poly-β-malic acid (PMLA), poly-β-alkanoic acids, polycarbonates, polyorthoesters, polyphosphates, poly(ester anhydrides), and mixtures thereof.

The use of matrix polymers selected from the group consisting of polylactides (PLA), poly-L-lactide (PLLA), poly-DL-lactide (PDLLA), polyglycolide (PGA), poly(ε-caprolactone) (PCL), copolymers of glycolide, glycolide/trimethylene carbonate copolymers (PGA/TMC), lactide/tetramethylglycolide copolymers, lactide/trimethylene carbonate copolymers, lactide/d-valerolactone copolymers, lactide/ε-caprolactone copolymers, L-lactide/DL-lactide copolymers (PLDLA), glycolide/L-lactide copolymers (PLGA), polylactide-co-glycolide, lactide/glycolide/trimethylene carbonate terpolymers, lactide/glycolide/ε-caprolactone terpolymers, PLA/polyethylene oxide copolymers, poly(ε-caprolactone-DL-lactide) copolymers and combinations thereof may be preferred. The use of matrix polymers selected from the group of polylactide, poly(lactide-co-glycolide), poly(lactide-co- ε-caprolactone), polyglycolide (PGA), and poly(ε-caprolactone) (PCL) and combinations thereof may be particularly preferred.

In a preferred embodiment of the method, the ratio between the inherent viscosity of the matrix polymer in the solid polymer composite material and the inherent viscosity of the (virgin) matrix polymer as provided in b) is between 0.5 and 1.5, preferably between 0.8 and 1.2, most preferably between 0.9 and 1.1. The property of inherent viscosity (IV) is known in the art and can be determined by dilute solution viscosity measurements, e.g., according to method ASTM 2857 using a glass capillary viscometer.

In general, the inherent viscosity of the matrix polymer in the solid polymer composite material does not deviate too much from that of the matrix polymer before it is combined with the solvent.

It is preferred that the solvent comprises or consists of acetone, chloroform, dichloromethane, tetrahydrofuran and/or ethyl acetate. In all cases, there is a preference for a relatively pure solvent. Accordingly, it is considered preferred for the solvent to consist for at least 80 wt.% of acetone, chloroform, dichloromethane, tetrahydrofuran or ethyl acetate, in particular for at least 90 wt.%, more in particular for at least 95 wt.%, still more in particular for at least 98 wt.%, or even at least 99 wt.%. If the solvent comprises or consists of acetone, the anti-solvent is preferably selected from water, a C₁-C₆ alcohol, a water-acetone mixture, a water-C₁-C₆ alcohol mixture, and a water-acetone-C₁-C₆ alcohol mixture. If the solvent comprises or consists of chloroform or dichloromethane, the anti-solvent is preferably selected from a C₁-C₆ alcohol-chloroform mixture, a C₁-C₆ alcohol-dichloromethane mixture, a C₁-C₆ alcohol, or a mixture thereof. If the solvent comprises or consists of ethyl acetate, the anti-solvent is preferably selected from water, a C₁-C₆ alcohol, a water-acetone mixture, a water-C₁-C₆ alcohol mixture, a water-acetone-C₁-C₆ alcohol mixture, and acetone.

There are, of course, many (further) useful combinations of polymer, solvent and anti-solvent. Table 1 presents a list of preferred matrix polymers for the process according to the invention, along with the preferred choices for solvent and anti-solvent corresponding to said preferred matrix polymer. Although all combinations mentioned in Table 1 are considered to be working examples according to the invention, it is hereby stressed that this Table 1 is non-exhaustive in the sense that further combinations can be possible as well and that other polymers, solvents and/or anti-solvents can be used for the process of the invention.

**Table 1: Examples of preferred matrix polymer, solvent and anti-solvent combinations.**

| *Polymer* | *Solvent* | *Anti-solvent* |
|---|---|---|
| Poly(L-lactide) | Chloroform, dichloromethane | Alcohols |
| Poly(L,D-lactide) | acetone, tetrahydrofuran, ethyl acetate, ethyl lactate, N-methyl-2-pyrrolidone, dimethyl sulfoxide, dimethylformamide chloroform, dichloromethane | Water, alcohols Alcohols |
| Polyglycolide, glycolide/L-lactide copolymers | Hexafluoroisopropanol | Water |
| Polycaprolactone | acetone, tetrahydrofuran, ethyl acetate, ethyl lactate, N-methyl-2-pyrrolidone, dimethyl sulfoxide, dimethylformamide | Water, alcohols |
| | chloroform, dichloromethane | Alcohols |
| Copolymers and terpolymers comprising glycolide, lactide, caprolactone, valerolactone, trimethylene carbonate, tetramethylglycolide | acetone, tetrahydrofuran, ethyl acetate, ethyl lactate, N-methyl-2-pyrrolidone, dimethyl sulfoxide, dimethylformamide | Water, alcohols |
| | Chloroform, dichloromethane | Alcohols |
| Polyhydroxybutyrates (PHB), PHB/b-hydroxyvalerate copolymers (PHB/PHV), poly-b-hydroxypropionate (PHPA) | Chloroform, dichloromethane | Alcohols |
| Methyl methacrylate-N-vinyl pyrrolidone copolymers | Acetone, chloroform, methanol, diethyl ether, ethyl acetate | Hexane |
| Polyvinyl alcohol (PVA) | Water | Acetone |
| Polyorthoesters | Acetone, tetrahydrofuran, ethyl acetate | Water, alcohols |
| Poly(ester anhydrides) | Acetone, tetrahydrofuran, ethyl acetate | Water, alcohols |

The method of the invention can be expanded with one or more additional steps after the solid polymer composite has been obtained in step d). A preferred example of such a step, which may be denoted as e), comprises shaping the solid polymer composite as obtained in step d), e.g., to form a medical device, e.g., an implantable device. Examples of suitable shapes are tapes, strands, (cannulated) rods, tubes, pellets, granules, or filaments. Other examples of implantable devices such as orthopedic implants are , screws, nails, wires, pin wires, anchors, cables ties, or wire ties, plates and screw systems, and external fixators or filament. These are just a few examples of orthopedic implants based on polymer composites. The choice of material will depend on the specific requirements of each device, such as biocompatibility, mechanical strength, and durability.

It is hereby understood that shaping implies handling the polymer composite in such a manner that the composite end-product has a certain desired shape. This can be achieved in any known manner, including pressing, such as pressing into a form, heat forming, cutting, slitting, calendaring and/or rolling. It is hereby understood that multiple operations can be combined, such as multiple rolling steps resulting in a further thinning of the rolled tape and optionally cutting the rolled tape to obtain strips. Other shaping methods include for example injection molding, compression molding, stereolithography, extrusion or thermoforming. In case of injection molding the polymer composite is melted and injected into a mold, which is then cooled and solidified to shape the device. In case of compression molding the polymer composite is placed in a mold and subjected to high pressure and heat, which fuses the material into the desired shape. Stereolithography is a process whereby a laser is used to selectively cure the polymer composite material layer by layer to create a 3D shape. Extrusion is a process whereby the polymer composite is melted and forced through a die to create a long, continuous shape, which can then be cut to size. Thermoforming is a process whereby the polymer composite is heated and formed into a mold using vacuum or pressure. The choice of shaping method will depend on factors such as the desired shape, production volume, and material properties of the polymer composite being used.

In the method of the invention, the solvent may preferably be allowed to partially evaporate before addition of the anti-solvent. This provides the advantage that a reduced amount of anti-solvent may be needed to remove the solvent from the composite. Hence, the method of the invention preferably comprises a step c2) evaporating at least part of the solvent from the mixture that has been applied onto the plurality of glass fibers.

The step d) of removing the solvent from the mixture using an anti-solvent to obtain the solid composite material can be carried out by contacting the glass fiber provided with the mixture with an antisolvent, e.g., by passing the glass fiber through a bath of anti-solvent. Other methods will be evident to the skilled person. The solid composite material will be recovered from the bath, and remaining anti-solvent can by removed, e.g., through evaporation.

The glass fibers may suitably have a diameter of 5 - 30 µm, preferably 6 - 20 µm, when measured using ASTM D1577-01 option C. In one embodiment, the glass fibers used in the present invention preferably have a diameter below 30 µm, in particular below 25 µm, more in particular below 20 µm in some embodiments below 15 µm.

In one embodiment, the glass fibers are applied in the form of a glass fiber bundle. In one embodiment, the glass fiber bundle has a linear density of between 5 and 5000 tex, preferably between 20 and 1300 tex, more preferably between 25 and 750 tex, still more preferably between 50 and 500 tex, even more preferably between 70 and 300 tex, for the total bundle, as measured according to ASTM D1577-01 option A. i, as measured according to ASTM D1577-01 A. The term linear density corresponds to the weight of a certain length of the glass fiber bundle and the unit tex corresponds to the weight in grams per 1000 meters of fiber.

Glass fiber bundles that may be used in the present invention preferably have at least 50 fibers, preferably at least 100, more preferably at least 150, and even more preferably at least 200 fibers. As a maximum, a value of 5000 fibers may be mentioned, more in particular 3000.

In one embodiment glass fiber bundles are used in the present invention in which the coefficient of variation of the diameter of the glass fibers in the glass fiber bundle is at most 15%, in particular at most 10%. The coefficient of variation may be at most 8%, or at most 6%. It has been found that in some embodiments the coefficient of variation may be at most 3%, which is a measure of a very even diameter indeed. The coefficient of variation is determined as follows: For 30 individual glass fibers in a glass fiber bundle, the diameter is determined. The average diameter and the standard deviation are calculated. The coefficient of variation is the standard deviation divided by the average diameter, expressed in %.

In a preferred embodiment, the glass fibers are bioactive glass fibers. Bioactive glass fibers are known in the art and have been designed to elicit or modulate biological activity. Bioactive material often is surface-active material that can interact with mammalian tissue. Bioactive glass may further be designed to leach ions or other chemicals resulting in osteoconductive, osteoinductive, anti-infective and/or angiogenic benefits.

The glass fibers can be silica-based, boron-based or phosphorus-based glass fibers. Preferably the glass fibers are silica-based.

The glass fibers used in the present invention preferably have a composition comprising network formers and network modifiers, wherein the molar ratio between the network formers and network modifiers is between 1 and 4, preferably between 1.5 and 3.5, more preferably between 2 and 3. It has been observed that ratios according such a range, and in particular one of the preferred ranges leads to a good glass quality, which is particularly suitable for fiber formation.

The glass fibers preferably comprise network formers which are selected from oxides of silicon, such as silica (SiO₂), oxides of boron, such as diboron trioxide (B₂O₃) and boron suboxide (B₆O), and oxides of phosphorous, such as phosphorous trioxide (P₂O₃) and phosphorus pentoxide (P₂O₅ or P₄O₁₀). The glass fibers preferably comprise network modifiers which are selected from oxides of sodium, such as Na₂O or Na₂O₂, oxides of magnesium, such as MgO, and oxides of calcium, such as CaO. More preferably, the glass fibers comprise several network formers and several network modifiers.

The glass fibers preferably have a composition comprising
50 - 75 wt.% of SiO₂, in particular 55-75 wt.%, more in particular 60-75 wt.%,
0 - 15 wt.% of B₂O₃,
0.5 - 5 wt.% of P₂O₅, in particular 0.5-4 wt.%, more in particular 0.5-3 wt.%,
5 - 20 wt.% of Na₂O,
0 - 25 wt.% of CaO, in particular 2-25 wt.%, more in particular 5-25 wt.%.
0 - 10 wt.% of MgO,
0 - 1 wt.% of Li₂O,
0 - 15 wt.% of K₂O, in particular 0-10 wt.%, more in particular 0-4 wt.%,
0 - 4 wt.% of SrO,
0 - 5 wt.% of Al₂O₃, and
0 - 5 wt.% of Fe₂O₃
(not calculating sizing, if present).

In one embodiment, the glass fibers (not calculating sizing, if present) have a composition comprising 60 - 75 wt.% of SiO₂, 0 - 15 wt.% of B₂O₃, 0.5 - 3 wt.% of P₂O₅, 5 - 20 wt.% of Na₂O, 5 - 25 wt.% of CaO, 0 - 10 wt.% of MgO, 0 - 1 wt.% of Li₂O, 0 - 4 wt.% of K₂O, 0 - 4 wt.% of SrO, 0 - 5 wt.% of Al₂O₃, and 0 - 5 wt.% of Fe₂O₃.

In one embodiment, the glass composition comprises less than 10 wt.% of B₂O₃, in particular less than 5 wt.%. In one embodiment, the glass composition comprises 7-20 wt.% Na₂O. In one embodiment, the glass composition comprises 2-8 wt.% MgO. In one embodiment, the glass composition comprises 5-15 wt.% CaO.

The glass fibers preferably have a tensile strength of 1000 - 3000 MPa, preferably between 1200 - 2500 MPa, more preferably 1400 - 2200 MPa, as measured by tensile testing according to DIN EN ISO 5079.

The glass fibers preferably have an elastic modulus between 20 - 100 GPa, preferably between 35 - 85 GPa, more preferably between 50 - 70 GPa as measured by tensile testing according to DIN EN ISO 5079 "Determination of breaking force and elongation at break of individual fibres" (ISO 5079:2020).

Attractive glass fibers and glass fiber bundles for use in the present invention are described in a patent application with the same applicant, inventors, and filing date as the present application, with the title "Resorbable and biocompatible glass fiber bundle having a well-defined diameter and process for making such", the text of which is incorporated herein by reference in its entirety.

In a preferred method, multiple mixtures comprising a matrix polymer and a solvent are applied onto the glass fibers. It is understood that this includes the case where the same mixture is applied more than once, the case that multiple mixtures having the same matrix polymer in varying concentrations are applied successively and/or the case that different mixtures having a different matrix polymer and/or a different solvent are applied successively. Ideally the multiple mixtures are applied by performing b) and c) multiple times in a subsequent fashion (e.g. 2, 5, 10 times).

It is thus understood that the invention may relate to a method for obtaining a solid composite comprising a plurality of biocompatible and resorbable glass fibers which are embedded in a biocompatible and resorbable matrix polymer, comprising the steps of:
a) providing a plurality of biocompatible and resorbable glass fibers,
b) providing a first mixture comprising a first biocompatible and resorbable matrix polymer that is compatible with the glass fibers and a first solvent,
c) applying the first mixture onto the plurality of glass fibers,
d) optionally removing the first solvent from the first mixture using a first anti-solvent,
e) optionally allowing the glass fibers coated with the first matrix polymer to dry at least partially,
f) providing a second mixture comprising a second biocompatible and resorbable matrix polymer that is compatible with the glass fibers and/or the first biocompatible and resorbable matrix polymer and a second solvent,
g) applying the second mixture onto the plurality of glass fibers, and
h) removing the solvent from the mixture using an anti-solvent to obtain the solid composite.

It is understood that the anti-solvent in step h) needs to be an anti-solvent for at least the second mixture and preferably is an anti-solvent for the first and the second mixture. If step d) is omitted, the anti-solvent of step h) preferably is an anti-solvent for the first and the second mixture. It is further understood that the first and second matrix polymer may be the same polymer or different polymers. Also, the first and the second solvent may be the same solvent of different solvents.

It is noted that, if so desired, the solvent may be removed partially from the system before application of the anti-solvent by evaporation, between steps c) and d) and/or between steps g) and h).

In a preferred embodiment, the polymer concentration in the second mixture is higher than the polymer concentration in the first mixture. The polymer concentration is the second mixture is preferably at least 1.5 times that of the first mixture, preferably at least 2 times that of the first mixture and, more preferably at least 2.5 times that of the first mixture.

A particular advantage of the method of the invention is that it allows to produce composites having a lower Yellowness Index (YI) compared with methods according to the current art. A preferred method is hence a method wherein the difference in Yellowness Index between the matrix polymer provided in step b) and the composite obtained is less than 20, preferably less than 10, more preferably less than 5, and even more preferably less than 2. The term YI is known in the art and is preferably determined according to ASTM E313. Yellowness is often caused by the use of heat, which is not required in the method according to the invention.

Preferably the composite material has a Yellowness Index (YI), as measured according to ASTM E313, of less than 30, preferably less than 20, more preferably less than 15, and even more preferably less than 10.

Another advantage of the invention is that it allows to produce composites with a very high glass fiber content. The method of the invention is hence preferable for obtaining a solid composite comprising at least 60 wt.% of a plurality of biocompatible and resorbable glass fibers, preferably at least 70 wt.% of a plurality of biocompatible and resorbable glass fibers, more preferably at least 80 wt.% of a plurality of biocompatible and resorbable glass fibers and even more preferably at least 90 wt.% of a plurality of biocompatible and resorbable glass fibers.

A further advantage of the method according to the invention is that the number of voids and/or air bubbles at the glass / matrix interface is lower compared to conventional melt techniques. These voids and/or air bubbles scatter light and may thus give the composite an off-white appearance and/or a whiteish color. As the voids or bubbles often form along the glass fibers, this results in the appearance of white streaks. It is speculated that the melted matrix polymer in conventional melt techniques does not sufficiently wet the glass fiber surface causing the formation of voids and bubbles. The solvent method of the invention does not suffer from this drawback and produces a composite absent of voids or bubbles, or at least having a very low number of voids or bubbles, resulting in a composite with a more homogeneous appearance.

The method of the invention allows to incorporate additives, such as bioactive additives, into the composite. Hence in a preferred embodiment the method according to the invention further comprises embedding an additive, for instance an active pharmaceutical ingredient (API) or a mineral ingredient, in the composite.

The invention further relates to a composite obtainable by the method of the invention. Also, the invention relates to a composite obtained by the method of the invention.

The invention further relates to a composite, comprising a plurality of glass fibers embedded in a polymer matrix that is compatible with the plurality of glass fibers, wherein the glass fibers and the polymer matrix are biocompatible, resorbable and preferably bioactive, wherein the composite has a monomer content lower than 1 wt.%. Preferably the monomer content is lower than 0.5 wt.%, more preferably lower than 0.2 wt.%, and even more preferably lower than 0.1 wt.%. The monomer content is calculated on the amount of polymer matrix. These monomers can originate from different sources, but an important source is degradation due to high temperatures needed to prepare the composite (i.e. high temperatures needed to obtain a polymer melt suitable for extrusion).lt is beneficial to have a low monomer content as high monomers levels indicate a loss of strength as well as other mechanical properties. This is especially relevant for the case of implants, where the composite is placed inside a mammalian, preferably human, body for a prolonged period. It is an attractive feature of the method of the invention that it makes it possible to manufacture composites with a low monomer content. On the one hand this is caused by the fact that thermal degradation is avoided because the polymer does not have to reach melting temperature. On the other, the use of a solvent - anti-solvent combination in itself reduces the presence of monomers in the polymer matrix, as the extraction of residual monomers from composite into the solvent and/or anti-solvent will be an additional purification step of the matrix polymer.

Preferably the plurality of glass fibers in the composite are at least partly coated with a sizing layer, in particular a sizing comprising a compatibilizer which is compatible with the matrix polymer. Hence, in a preferred embodiment the resorbable glass fibers compatible with the matrix polymer are resorbable glass fibers, having a sizing layer on their surface that is compatible with the matrix polymer. An attractive sizing and method for its application are described in a patent application with the same applicant, inventors, filing date, and priority date as the present application, with the title "Resorbable glass fiber coated with a sizing and method for preparing such", the text of which is incorporated herein by reference in its entirety. Further sizing compositions are known in the art, and require no further elucidation here.

In one embodiment, at least 10% of the structural units of the compatibilizer are identical to the structural units of the matrix polymer. This is one way to ensure good compatibility of the glass fiber with the matrix material. In one embodiment, at least 20% of the structural units of the compatibilizer are identical to the structural units of the matrix polymer, or at least 40%, or at least 60%. In one embodiment, both the compatibilizer and the matrix polymer comprise at least 20 wt.% of lactide units, in particular at least 30 wt.%. In one embodiment, both the compatibilizer and the matrix polymer comprise at least 20 wt.% of caprolactone units, in particular at least 30 wt.%. In another embodiment, the compatibilizer and the matrix polymer comprise at least 20 wt.% of glycolide units, in particular at least 30 wt.%.

Preferably the composite comprises an additive, for instance an active pharmaceutical ingredient (API) or a mineral ingredient, embedded within the composite.

Preferably the composite has a low number of air bubbles and/or voids. It is understood that air bubbles and voids inside the composite give the composite a milky and whiteish appearance. Hence preferably the composite according to the invention is translucent and more preferably transparent. It is hereby understood that translucency and transparency are optical properties and relate to the passage of visible light, whereby visible light is light having a wavelength within the visible spectrum of about 380 to about 750 nanometers. In one embodiment, the composite has a transmission for visible light of at least 50%, in particular at least 70%, more in particular at least 80%. It is preferred that the composition is radiopaque, meaning that it blocks X-rays. It is understood that X-rays refers to high-energy radiation having a wavelength from about 10 pm to 10 nm. That the composite is radiopaque can be achieved by proper selection of the glass fibers. A radiopaque composite is an attractive choice for implantable medical devices because it allows monitoring of the degradation of the composite through X-ray in-vivo.

In the composite, the glass fibers or glass fiber bundles may be present as continuous fibers and/or a chopped fibers. Chopped fibers, if used, generally have a length in the range of 1 mm to 50 mm, in particular 1 mm to 25 mm, more in particular 1 mm to 20 mm, even more in particular 2 mm to 10 mm, most in particular below 4 mm and above 1 mm.

In one embodiment, the medical device comprises an unidirectional composite tape. Glass-fiber-based tapes are known in the art. They comprise a biodegradable polymer and a plurality of unidirectionally aligned continuous glass fibers, with the fibers being aligned in the length direction of a tape. The tape generally has a width which is at least 2 times the thickness of the tapes, in particular at least 5 times, more in particular at least 10 times. The length of the tape generally is at least 10 times the width of the tape, in particular at least 100 times. The thickness of the tape is preferably less than 0.3 mm, more preferably less than 0.2 mm and even more preferably less than 0.15 mm. The length of the tape is preferably at least 1 m, more preferably at least 5 m, and even more preferably at least 10 m. The third dimension (i.e. the width) is preferably between 0.5 and 10 cm, more preferably between 0.8 and 5 cm, and even more preferably between 1 and 2.5 cm. Composite tapes can be obtained, e.g., by spreading the fibers of a glass fiber bundle, contacting the glass fibers with a polymer matrix in the liquid phase, and solidifying the matrix. Unidirectional composite tapes preferably have a matrix content of 2-40 wt.%, in particular 5-30 wt.%, more in particular 5-25 wt.%, in specific embodiments 10-20 wt.%. The preferences given elsewhere for the nature of the matrix and the nature of the glass fiber also apply here.

In one embodiment, the medical device comprises a plurality of layers, wherein one or more layers comprise one or more composite tapes.

Thus, in one embodiment, the composite is shaped in the form of an unidirectional composite tape, as discussed above. In an alternative embodiment, the composite material is shaped as a strand, rod, pellet or granule. A strand/rod is a round-shaped fiber reinforced polymer with a diameter that is preferably between 5 - 50 mm. Pellets are small length-wise sections of a rod having a diameter of about 5 - 50 mm and granules are small particles having a diameter of about 1 - 10 mm. The dimensions of the pellets or granules may be adapted to match the dimensions of feeding screws used in polymer processing techniques, for example injection molding.

Preferably the inherent viscosity of the matrix polymer in the composite material is between 1.5 and 4.0 dL/g, preferably between 1.8 and 3.0 dL/g, more preferably between 2.0 and 3.0 dL/g.

In general, the polymer composite material has a Young's modulus (tested per ASTM D7264/D7264M) which is higher than the Young's modulus of the matrix polymer, preferably between 110% and 1000% compared to the matrix polymer, more preferably between 200% and 600%, most preferably between 300% and 500%.

Preferably the glass reinforced polymer composite material has a mechanical strength according to a three-point bend test (tested per ASTM D7264/D7264M) higher than the strength of the matrix polymer, preferably at least 110% compared to the matrix polymer, preferably at least 200% compared to the matrix polymer, most preferably at least 400% compared to the matrix polymer when the applied force is perpendicular to the reinforcement fibers.

Preferably the polymer composite has a glass fiber content, calculated on the total of glass and polymer of between 5 and 95 wt.%, preferably between 10 and 90 wt.%, more preferably between 15 and 80 wt.%, even more preferably between 20 and 70 wt.%, most preferably between 30 and 60 wt.%. Alternatively, the polymer composite preferably has a glass fiber content of at least 60 wt.%, more preferably at least 70 wt.%, even more preferably at least 80 wt.% and even more preferably at least 90 wt.%.

The composite according to the invention may comprise further components in addition to glass and polymer, e.g., up to 30 wt.%, up to 20 wt.%, or up to 10 wt.%. Examples of further components may be hydroxy-apatite and calcium phosphate, such as tricalciumphosphates (TCP), e.g. β-TCP.

The glass reinforced polymer composite material according to the invention is particularly suited for use in a medical device, e.g., a medical implant. The invention hence also relates to a medical device comprising the composite material according to the invention. Preferably this medical device is an implant or a scaffold comprising the composite according to the invention.

As will be evident to the skilled person, different embodiments of the present invention can be combined unless they are mutually exclusive.

All percentages used herein are weight percentages, unless specified otherwise.

When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

The invention will be elucidated with reference to the following examples, without being limited thereto or thereby.

### Examples

### Measurement techniques

### Inherent viscosity

Inherent viscosity (IV) is a measure for the average molecular weight of polymers derived from the dilute solution viscosity of polymers. It is based on the comparison of the flow time of a sample solution through a narrow tube (i.e. an Ubbelohde) to the flow time of a known pure solvent through an equally sized tube. This is according to ASTM 2857.

To determine the IV, three solutions of 2, 1, and 0.5 g/dL of matrix polymer are prepared from composite samples. These solutions are prepared by dissolving composite samples in chloroform and subsequently filtering the solutions through a 0.2 µm filter to remove the glass fibers. The polymer concentrations are corrected for the glass content as determined using TGA. A blank solution is also prepared containing only solvent. The measurement tube was rinsed with filtered sample solution using the 0.2 µm filter. Subsequently it was filled with the filtered sample solution up to between the 2 lines and placed in a water bath set to 25°C. The flow time t₀ of the sample is then measured and corrected with the Hagenbach correction tₕ to obtain t_{g}. Based on the t_{g} of the sample and that of the blank, the relative viscosity was calculated using ηᵣₑₗ = t_{g,sample}/t_{g,blank}. The inherent viscosity ηᵢₙₕ was calculated using ηᵢₙₕ = ln(ηᵣₑₗ)/C, with C being the concentration of the sample in g/dL. IV accordingly has the unit dL/g.

The resulting inherent viscosity and sample weight is recorded on the appropriate forms in the batch record. The same method is repeated for the other solutions.

### Yellowness Index

The yellowness index (YI) was measured using a Chromometer CR-410 from Konica Minolta according to ASTM D1925. The measurements were performed in triplicate. The instrument was calibrated with a standard calibration plate (No. 12133209) with Y = 85.6, X = 0.3150 and y = 0.3212.

### Tape dimensions

The width was measured using a digital microscope. An image of the tape is taken and then width was measured using Zen core version 2.5 software or Keyence microscope's software. The thickness was measured by using a wide-faced screw gauge (or a caliper).

### Three-point bending test

Thin layers of pre-made composite tapes were stacked together in a uniform direction to make samples for testing. The number of tape layers depends upon the required thickness of the intended composite. In a rectangular shaped (100mm x 100mm) compression mold, these composite layers were then heated above the polymer's melting point (190-200 °C), pressed at 12kN load for 5 minutes, and then quenched to obtain a composite plaque.

The composite plaque was then cut into rectangular coupons and tested according to ASTM D7264/D7264M-15. Span to thickness: 16:1 ratio was used. Standard width: 13mm with the specimen length being about 20% longer than the support span (10% each side).

### Loss on ignition via thermogravimetric analysis (TGA)

The loss on ignition technique (LOI) is a technique suitable to determine the matrix content of glass fiber samples. In a LECO 701 macro thermogravimetric analyzer (TGA), the matrix content on 1 ± 0.2 g of sample was determined. The test began by logging in the samples into the software followed by the determination the initial (empty) crucible weights (with lids). Once the initial weights have been taken, the samples were added to the crucibles in their respective position and the equipment would determine the weight of the samples. The instrument subsequently heats up to 110 °C which is maintained for 30 minutes. After said 30 minutes the equipment was heated to 565 °C at a rate of 25 °C/min and is held at that temperature for 2 hours. The weights were determined at regular time intervals whilst the instrument was running and the % mass loss for each step is calculated and reported. The first (110 °C step) provided the moisture content in the sample without burning any sizing from fibers. The higher temperature (565 °C) provided the matrix content by burning off the matrix polymer from the glass fibers.

### Gas chromatography (GC)

Polymer from a composite sample (equivalent to 300 mg polymer based on TGA data) was dissolved in 25 mL chloroform and then filtered through a 0.2 µm filter. An Agilent 7820A GC system equipped with a FID detector with autosampler (7650A) with an Agilent DB-225 capillary column was used. The temperatures for the injector, detector and column oven were set at 200 °C. High-purity nitrogen (99.999 % pure) was used as the carrier gas. The injection volume was 1.0 µL with an auto-sampler. The injected sample solution is separated using a capillary column and detected using a flame ionization detector (FID). The peak area is a measure for the concentration.

### Relative gel permeation chromatography (GPC):

A gel permeation chromatographer (Agilent 1200 Series) was used to measure the relative number-averaged and weight-averaged molecular weight (Mₙ and M_{w}, respectively) of the compatibilizer against polystyrene standards. The GPC consisted of a guard column and 2 PL gel mixed D columns and an evaporative light scattering detector (ELSD). HPLC Grade chloroform stabilized with ethanol (Biosolve) was used as the mobile phase at a flow rate of 1 mL/min and the measurement was conducted at 35 °C. Samples of 15 mg of compatibilizer were dissolved in 15 mL of chloroform and then filtered through 0.45 µm filter prior to GPC analysis. The GPC system was calibrated using narrow polystyrene standards.

### Comparative Example A - manufacture of composite tapes using a polymer melt

Multi-filament glass fiber bundles, type NX-8, with a composition of 67.8 wt.% SiO₂, 1.5 wt.% P₂O₅, 2.3 wt.% B₂O₃, 9 wt.% CaO, 5.4 wt.% MgO and 14 wt.% Na₂O were prepared by melt spinning using a 204 tip platinum bushing set-up, followed by in line dip-coating with a dispersion comprising epoxy silane and degradable and acid functionalized thermoplastic based sizing which was dried and cured onto the glass fibers from 35-140 °C. There were 204 fibers (also indicated as filaments) per fiber bundle, the fibers having an average diameter of approximately 11 micrometers, as confirmed by SEM.

A polymer melt was prepared from Purasorb PLDL 7028 polylactide (ex. Purac America Inc., Lenexa, KS), dried at room temperature under vacuum before use. The inherent viscosity of the polylactide copolymer chips was measured to be 2.8 dL/g.

A plurality of these multifilament, bioresorbable NX-8 glass fiber spools were aligned and individually wound from horizontal axes in a creel at a rate of 1-3 m/min via pultrusion, towards a twin belt puller (with PUR belts), providing the necessary force to pull the fibers through the polymer melt and unwind the spools in the creel.

The polymer melt was provided at a feeding rate of 500 - 2000 g/hour by means of a twin screw extruder, feeding an electrically heated crosshead die with the PLDL 7028 melt at a maximum temperature of 235°C.

After the creel and before the crosshead die, the glass fiber bundle was passed under mild tension over horizontal pins (set temperature 275°C), in order to equalize the tension and spread the warp, and then entered into the feed side of the crosshead die.

In the crosshead die, hot polymer melt is contacted with the glass fiber warp, while it passed over and under stationary horizontal pins for additional spreading and wetting. The prepreg was subsequently passed through a slit die of desired shape (circular for strand/pellet and rectangular for tape) and geometry. For tape as the end product, the impregnated tape from the shaping die was calendared under mild pressure. The composite tape or strand was then air-cooled prior to being picked up by the puller. After the puller, the consolidated tape or strand was collected on spools. The circular strand was pelletized using a strand pelletizer after passing through the forming die and through the puller. Various experiments were carried out using this method to investigate its possibilities. The results obtained thereby are listed in Table 2.

### Example 1 - manufacture of composite tapes with poly(L-lactide) matrix

Purasorb PLDL 7020 (Polylactide copolymer with 70 mol.% L-lactide and 30 mol.% D-lactide having an inherent viscosity of 2.0 dL/g, ex. Purac America Inc., Lenexa, KS) was dissolved in three mixing vessels at three different concentrations (7, 12 and 20 %w/w, respectively). Resorbable glass fibers, coated with a covalently bonded sizing consisting of epoxy silane reacted with an acid-functional lactide-eps.-caprolactone copolymer (Mn 5.6, Mw 8 kg/mol as measured by relative GPC), with the target composition of SiO₂ 67.8 wt.%, P₂O₅ 1.5 wt.%, B₂O₃ 2.3 wt.%, CaO 9 wt.%, MgO 5.4 wt.% and Na₂O 14 wt.% were pulled from a creel. Total target linear density of the combined fiber bundle was 2500 tex, according to ASTM D1577-01 A, with an average fiber diameter of 14.7 micrometer.

The glass fibers were guided from the creel via a horizonal reed and threaded through the various sections of the composite line having a chain of aligned modules. This line comprised baths which were supplied with either acetone or polymer solution in acetone. In the first bath the excess and unreacted sizing was washed away and the sized glass fibers got spread with the help of concave and convex pins. A nicely spread, wide fiber warp was achieved. This warp of fibers was then introduced into the low concentration (7 wt.%) polymer solution bath where all the fibers get coated with a thin layer of polymer solution. This coated warp is then air dried before introduction into the second, medium concentration (12%) polymer solution. The warp picks up more polymer from the second bath and gets air dried subsequently. At this point, the so-called prepreg was collected onto spools and vacuum dried overnight. The next day, the prepreg was loaded onto a creel and pulled through a high concentration (20%) polymer solution bath. The exit of this bath was equipped with a rectangular die to aid formation of the prepreg into a desired flat shape. The desired shaped product with desired polymer/glass fiber ratio was subsequently passed through a finishing section comprising three solvent exchanger baths. The first anti-solvent bath contained a mixture of 70% acetone and 30% water at -10°C, the second anti-solvent bath was 50% acetone/50% water at -10°C and the third anti-solvent bath was 100% water at a temperature of 50°C. The product spent at least 90 seconds in each bath. After the third bath the wet product was collected onto spools lined with a polypropylene mesh. The spools were then dried in vacuo for 3 days to remove residual acetone and water. After 3 days of drying, the product was hot-calendared and slit to achieve desired specification (width and thickness) tape. A radiopaque and transparent composite was obtained. Various experiments were carried out using this method to investigate its possibilities. The results obtained thereby are listed in Table 2.

As can be seen from Table 2, the method of the invention allows for much higher fiber contents compared to the prior art method. Furthermore, it is shown that the drop in IV during production is much lower for the composite of Example 1 than for the comparative example. Also, the monomer generation is lower for the composite of Example 1. It is hence concluded that there is less degradation of the matrix polymer during fabrication process of the composite when the method of the invention is used compared to the known method. In addition, it has been found that the spreading of the matrix polymer around the glass fibers is improved which implies a larger contact area between the glass fibers and the matrix and fewer voids, air bubbles and white streaks. The larger contact area means that the load transfer in the composite made is improved which is useful for making stronger composites. The lower amount of air bubbles, voids and white streaks adds to the transparency of the composite as air bubbles scatter light and cause a milky appearance.

### Example 2 - manufacture of composite tapes with poly(L-lactide) matrix

Purasorb PL 24 (Poly(L-lactide) homopolymer with an inherent viscosity of 2.4 dL/g) was dissolved in a mixing vessel at 10% (w/w) in chloroform until completely dissolved. One single bundle of resorbable sized glass fibers with the target composition of SiO₂ 67.8 wt.%, P₂O₅ 1.5 wt.%, β2O₃ 2.3 wt.%, CaO wt.9%, MgO 5.4 wt.% and Na₂O 14 wt.% was pulled from a feeding creel. The fibers were coated with epoxy silane and acid-functional lactide ε-caprolactone copolymer (Mn 5.6, Mw 8 kg/mol as measured by relative GPC) -based sizing covalently bonded onto glass fibers surface. The linear density of the bundle was 140 tex (or g/km), which corresponds with an average fiber diameter of 11 micrometers. The fibers were threaded through the various sections of a custom-made reel-to-reel applicator consisting of various modules including a polymer and an anti-solvent (ethanol) bath. The polymer bath was filled with the (10 wt. %) polymer solution in chloroform. In the polymer bath the glass fiber got spread with the help of static pins and impregnated with matrix polymer with the help of submerged rollers. The narrow and flat prepreg strand was then redirected into the anti-solvent bath via guide rollers which were also submerged in the anti-solvent (ethanol). The line speed was kept constant at 1 m/min throughout the experiment. After the anti-solvent (ethanol) bath the prepreg was non-sticky and almost dry before reaching the winder. The prepreg was then collected onto a spool. The collected spool was loaded onto let-off creel and the prepreg was threaded through the line for the second pass. The prepreg got coated with more polymer via rollers and then redirected into anti-solvent (ethanol) bath just like in the first pass. The flat strand was then collected onto a spool. The spools were finally dried in a vacuum oven to remove any residual solvent (CHCl₃) and ethanol.

The obtained tapes were subsequently analyzed, and their properties are listed in Table 3. From the monomer content and the comparison of the Mn before and after producing the tape, it can be concluded that the degradation of the matrix polymer during production was very low. Also the obtained tapes where very white indicating a low YI.

### Example 3 - manufacture of composite tapes with poly((L-D)lactide) matrix

Purasorb PLDL 7020 (Polylactide copolymer with 70 mol% L-lactide and 30 mol% D-lactide having an inherent viscosity of 2.0 dL/g) was dissolved in a mixing vessel at 10 wt.% in acetone. Powdered tri-calcium phosphate (milled α-TCP (lot# 13CAM02.97) from Cam Bioceramics) was gradually added to the polymer solution at 10 wt.%. A single bundle of sized, resorbable glass fibers with the target composition of SiO₂ 67.8 wt.%, P₂O₅ 1.5 wt.%, B₂O₃ 2.3 wt.%, CaO 9 wt.%, MgO 5.4 wt.% and Na₂O 14 wt.% was pulled from a creel. The fibers were coated with epoxy silane and acid-functional lactide-eps.-caprolactone copolymer (Mn 5.6, Mw 8 kg/mol as measured by relative GPC) -based sizing covalently bonded onto glass fibers surface. The average linear density of the bundle was 140 tex (g/km) with a corresponding average fiber diameter of 11 micrometer. The fibers were threaded through the various sections of a custom-made reel-to-reel applicator consisting of various modules including a polymer and anti-solvent bath. The polymer bath was filled with the polymer solution compounded with TCP in acetone. In the polymer bath the individual glass fibers got spread with the help of static pins and impregnated with matrix polymer/TCP with the help of submerged rollers. The narrow and flat prepreg strand was then redirected into the anti-solvent bath via guide rollers which were also submerged in ethanol. For the proof of concept, the anti-solvent bath consisted of 100% ethanol at room temperature. After the anti-solvent bath the prepreg was non-sticky and almost dry before reaching the winder. The prepreg was then collected onto a spool. The collected spool was loaded onto a let-off creel and the prepreg was threaded through the line for the second pass. The prepreg got coated with more polymer/TCP via rollers and was redirected into the anti-solvent (alcohol) bath just like in the first pass. The flat strand was collected again onto a spool. The spools were finally dried in a vacuum oven to remove any residual dichloromethane and ethanol. The line speed was kept constant at 1m/min throughout the experiment.

The obtained tapes were subsequently analyzed, and their properties are listed in Table 3. From the monomer content and the comparison of the Mn before and after producing the tape, it can be concluded that the degradation of the matrix polymer during production was very low. Also the obtained tapes where very white indicating a low YI.

**Table 2: Properties of the composites obtained in Comparative Example A and Example 1.**

| | *Comparative Example A* | *Example 1* |
|---|---|---|
| *Sizing content (TGA)* | 1.2 wt.-% on glass | 1.1 wt.-% on glass |
| *Maximum fiber weight fraction (TGA)* | 62.5%, beyond that fiber wetting was not good and fibers broke in the die | 90% of composite |
| *Drop in IV (dL*/*g)* | Dropped from 2.8 or 2.4 to as low as 1.79 | No drop in IV, remains around 2.0 |
| *Color* | tan to light brown | clear to white |
| *Yellowness Index (YI)* | 10.3 | 5.1 |
| *Monomer generation (GC)* | Up to 1.3% | <0.09%, lower than starting polymer |
| *Quality of impregnation (SEM)* | Not good (a lot of voids and dry fibers) | Improved spreading and low viscosity polymer solution resulted in well coated fibers |
| *Quality of fiber distribution (images)* | Bundled fibers, fiber and polymer rich section visible | Improved spreading resulted in better fiber distribution across the width of the tape |
| *Flexural strength* | 675MPa (average 50 wt.-% Fiber Loading) | 650MPa (average 42 wt.-% fiber loading) |
| *Flexural modulus* | 24GPa (average 50 wt.-% Fiber Loading) | 20GPa (average 42 wt.-% fiber loading) |
| *Tape width* | 11.9-13 mm | 13.8-20 mm |
| *Tape thickness* | 0.14-0.16 mm | 0.14-0.17 mm |

**Table 3: Properties of the composite obtained in Examples 2 and 3.**

| | *Example 2* | *Example 3* |
|---|---|---|
| *Sizing content* | 2.5 wt.% on glass | 2.5 wt.% on glass |
| *Polymer weight fraction from 1^{st} pass* | 42 wt.-% | 21 wt. % |
| *Polymer weight fraction from 2^{nd} pass* | 65 wt.-% | 23 wt.-% |
| *TCP content in polymer (TGA)* | Not applicable | 35 wt.-% |
| *TCP content* | Not applicable | 16 wt.-% |
| *Color* | White | white |
| *Monomer content (GC)* | 0.05 wt.- %, lower than starting polymer | 0.002 wt.-%, lower than the starting polymer |
| *Molecular weight (Mn) of raw polymer (GPC)* | 220 kg/mol | 230 kg/mol |
| *Molecular weight (Mn) of polymer matrix (GPC)* | 215 kg/mol | 225 kg/mol |
| *Average Tape width* | 20 mm | 16 mm |
| *Average Tape thickness* | 0.19 mm | 0.13 mm |

### Example 4 - Providing glass fibers with a sizing having polycaprolactone as compatibilizer, and incorporation of the coated fiber into a polycaprolactone matrix to form a polycaprolactone glass fiber-reinforced composite material, and further processing thereof to form a composite plaque

### 1. Preparation of Silane treated glass fibers

Hydrolyzed silane solution was prepared by dissolving 117g of 3-glycidyloxy-propyltriethoxysilane in 10kg of ASTM type II purified water at 50 degrees Celsius for 90 minutes. Hydrolyzed silane solution was then applied in-line via a kiss roller onto freshly drawn glass fibers as they are formed in a melt spinning process. The composition of the glass corresponded to that applied in Example 5. The resulting fiber bundle was wound onto a core for further processing. The wet glass fibers were air-dried and then vacuum-dried at room temperature to achieve moisture level under 1000ppm. The dried glass fibers were heat treated at 90°C for 4 hours to covalently bond silane with hydroxyl groups on the glass fiber surface.

### 2. Preparation of aqueous PC (IV 0.15) nanodispersion

40g of acid-functionalized polycaprolactone with an IV of 0.15 dL/g (measured according to ASTM 2857) and acid number of 39.4 mg KOH/g were dissolved in 1600g or acetone for 1 hour. After complete dissolution, triethylamine (TEA, from Sigma Aldrich), in 1:1 mol ratio to acid, was added to neutralize the acid end groups. After stirring for 15 minutes, the polymer solution was gradually added to ASTM type II purified water. After removing the acetone through evaporation for 24hr, an aqueous nanodispersion was obtained with a solids content of about 3 wt.%. The average particle size (z-average) as measured by DLS (Malvern ZetaSizer-nano) was found to be 37 nanometers, D90 was 39nm.

### 3. Preparation of PC (IV 0.15) sized glass fibers

The silane treated glass fibers (with an average diameter of 16 micron) obtained in step 1 above were further treated with compatibilizer polymer (PC, IV 0.15 dL/g) prepared in step 2 above by passing the bundle of fibers through the polymer nanodispersion bath in an off-line, reel-to-reel sizing applicator system in a continuous fashion. The line speed was 5m/min. The sized fibers were collected on a separate perforated stainless steel (SS) core. The fibers were subsequently vacuum dried at room temperature and then cured at 90°C for 16 hours to facilitate reaction between the acid end groups of compatibilizer and the epoxy groups of silane already covalently bonded to the glass surface. TEA present at the reaction site acted as a catalyst that was eventually removed by applying a vacuum.

Once cured, the PC-sized glass fiber sample was tested for presence of the PC (IV 0.15) compatibilizer sizing layer via TGA loss-on-ignition method. The results confirmed the presence of 1±0.5 wt.% sizing on glass fibers.

### 4. Manufacture of a composite tape comprising polycaprolactone polymer and fibers sized with polycaprolactone compatibilizer

10 wt.% solution of high molecular weight polycaprolactone (Purasorb PC17, , IV 1.7 dL/g, commercially available from Corbion Purac) was prepared in acetone at 50°C. One single bundle (576 filaments) of the PC-sized fibers obtained in step 3 was coated with the polymer by passing it through the polymer solution bath in a continuous reel-to-reel applicator system. The polymer solution bath was equipped with rollers and pins to spread fibers for better wet-out. The solvent was removed by passing the coated fibers through an anti-solvent (ethyl alcohol) bath at room temperature at a line speed of 2m/min, followed by air drying for 24 hours. After drying, the polymer-coated fibers appeared as thin and narrow tape. The coated and dried fibers were analyzed for fiber/polymer content via LECO, TGA701 loss on ignition method. The polymer content was 14.2 wt.%.

### 5. Composite plaques obtained by compression molding of the composite comprising polycaprolactone and coated glass fibers

The composite tape obtained under 4 was cut to 100mm long strips, and a predetermined amount was stacked and spread uniformly in the cavity of a preheated mold in a uniform direction to make samples for mechanical testing. Additional polymer (Purasorb PC17, Tm 60°C) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

### 6. Mechanical testing of the composite

The composite plaque from step 5 above was cut into rectangular coupons (50x13x1 mm; LxWxT) and tested for flexural properties (3-point-bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of 147±7.6 MPa (a 600% improvement from base polymer strength of 23MPa) and modulus of 6.46±0.68 GPa (58x improvement from 110MPa of base polymer) was measured for 6 samples tested. The tested samples were analyzed for fiber content via TGA loss on ignition method and the average fiber contents were found to be 50.4 wt.% of the composite.

### Example 5 - Providing glass fiber having poly(DL lactide) (PDL) as compatibilizer, and incorporation of the coated fiber into a poly(DL lactide) matrix to form a poly(DL lactide) glass fiber-reinforced composite material, and further processing thereof to form a composite plaque

Epoxy-functional, silane-treated glass fiber was prepared as described in Example 4 above.

30g of acid functionalized poly(DL lactide)(50/50) polymer with an IV of 0.4 dL/g and acid number of 4.1 mg KOH/g were dissolved in 1500g acetone for 1 hour. After complete dissolution, TEA, in 1:1.3 mol ratio to acid, was added to neutralize the acid end groups. After stirring the solution for 15 minutes the polymer solution was gradually added to purified ASTM type II purified water. After removing the acetone through evaporation for 24hr a ~3 wt.% aqueous dispersion was obtained. The average particle size (z-average) as measured by DLS (Malvern ZetaSizer-nano) was found to be 30 nanometers, D90 was 24nm.

The silane-treated glass fibers were further treated with compatibilizer polymer by passing the bundle of fibers through a bath of the polymer nanodispersion described above in an off-line reel-to-reel sizing applicator system in a continuous fashion. The line speed was 5m/min. The sized fibers were collected on a separate perforated SS core. The fibers were vacuum dried at room temperature and then cured at 90°C for 16 hours to facilitate reaction between -COOH end group of compatibilizer polymer and epoxy end of silane already covalently bonded to glass surface. TEA present at the reaction site acted as a catalyst which was eventually removed by applying a vacuum.

Once cured, the glass sample was tested for presence of sizing layer via TGA loss-on-ignition method. The results confirmed presence of 1±0.5 wt.% sizing on glass fibers.

10 wt.% solution of high molecular weight poly(DL lactide) (IV 2.0 dL/g) was prepared in acetone. One single bundle (576 filaments) of the sized fibers described above (with an average diameter of 16 micron) was coated with high molecular weight poly(DL lactide) by passing it through the polymer solution bath in a continuous reel-to-reel applicator system. The polymer solution bath was equipped with rollers and pins to spread fibers for better wet-out The solvent was removed by passing the coated fibers through an anti-solvent (ethylalcohol) bath at room temperature. The line speed was 1 m/min. The coated fibers were air dried for 24 hours. After drying the coated fibers appeared as thin and narrow tape. The coated and dried fibers were analyzed for fiber/polymer content via LECO TGA701 loss on ignition method. The polymer content was 15.4 wt.%.

The composite tape obtained was cut to 100mm long strips, and a predetermined amount was stacked and spread uniformly in the cavity of a preheated mold in a uniform direction to make samples for mechanical testing. Additional polymer (Purasorb PLDL7020) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

The composite plaque was then cut into rectangular coupons (50x13x1 mm; LxWxT) and tested for flexural properties (3-point bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of 491.3±38.6 MPa (a 446% improvement from base polymer strength of 110MPa) and modulus of 12.72±1.5 GPa (374% improvement from 3.4 GPa of base polymer) was measured for 6 samples tested. The tested samples were analyzed for fiber content via TGA loss on ignition method and the average fiber contents were found to be 52 wt.% of the composite.

### Example 6 - Providing glass fiber with a sizing having poly(DL lactide/glycolide) (PDLG) as compatibilizer, and incorporation of the sized fiber into a poly(DL lactide/glycolide) matrix to form a poly(DL lactide/glvcolide) glass fiber-reinforced composite material, and further processing thereof to form a composite plaque

Epoxy-functional, silane-treated glass fiber was prepared as described in Example 4 above.

30g of acid functionalized poly(DL lactide/glycolide) 50/50 from Corbion with an IV of 0.4 and acid number of 4.1 mg KOH/g was dissolved in 1500g acetone in 1 hour. After complete dissolution, triethylamine, in 1:1.3 mol ratio to acid, was added to neutralize the acid end groups. After stirring the solution for 15 minutes the polymer solution was gradually added to purified ASTM type II purified water. After removing the acetone through evaporation for 24hr a ~3 wt. % aqueous dispersion was obtained. The average particle size (z-average) as measured by DLS (Malvern ZetaSizer-nano) was found to be 46 nanometers, D90 was 46nm.

The silane treated glass fibers were further treated with the dispersion as described above by passing the glass fibers through a bath of the polymer nanodispersion in an off-line reel-to-reel sizing applicator system in a continuous fashion. The line speed was 5m/min. The sized fibers were collected on a separate perforated SS core. The fibers were vacuum dried at room temperature and then cured at 90°C for 16 hours to facilitate reaction between -COOH end group of compatibilizer polymer and epoxy end of silane already covalently bonded to glass surface. TEA present at the reaction site acted as a catalyst which was eventually removed by applying a vacuum.

Once cured, the glass sample was tested for presence of sizing layer via TGA loss-on-ignition method. The results confirmed the presence of 1±0.5 wt.% coating on glass fibers.

10 wt.% solution of high molecular weight poly(DL lactide/glycolide) 85/15 with an IV of 2.3 dL/g was prepared in acetone at 50°C. One single bundle (576 filaments) of the PDLG sized fibers described above (with an average diameter of 16 micron) was coated with PDLG85/15 by passing it through the polymer solution bath in a continuous reel to reel applicator system. The polymer solution bath was equipped with rollers and pins to spread fibers for better wet-out The solvent was removed by passing the coated fibers through an anti-solvent (alcohol) bath at room temperature. The line speed was 2m/min. The coated fibers were air dried for 24 hours. After drying the coated fibers appeared as a thin and narrow tape. The coated and dried fibers were analyzed for fiber/polymer content via LECO TGA701 loss on ignition method. The polymer content were 32.6 wt.%.

The composite tape obtained was cut to 100mm long strips, and a predetermined amount was stacked and spread uniformly in the cavity of a preheated mold in a uniform direction to make samples for mechanical testing. Additional polymer (Purasorb PLDG85/15) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

The composite plaque was cut into rectangular coupons (target 50x13x1 mm ; LxWxT) and tested for flexural properties (3pt bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of 770.7±131 MPa (a 770% improvement from base polymer strength of 100MPa) and modulus of 23.9±5.4 GPa (570% improvement from 4.2 GPa of base polymer) was measured for 6 samples tested. The tested samples were analyzed for fiber content via TGA loss on ignition method and the average fiber contents were found to be 55.7 wt.% of the composite.

## Claims

1. Composite, comprising a plurality of glass fibers in a polymer matrix, compatible with the plurality of glass fibers, wherein the glass fibers and the polymer matrix are biocompatible, resorbable and preferably bioactive, wherein the composite has a monomer content lower than 1 wt.%, preferably lower than 0.5 wt.%, more preferably lower than 0.2 wt.%, even more preferably lower than 0.1 wt.%, calculated on the polymer matrix.

2. Composite according to claim 1, wherein the composite has a Yellowness Index (YI) measured according to ASTM E313 lower than 30, preferably lower than 20, more preferably lower than 15, most preferably lower than 10, and/or wherein the composite is translucent and preferably transparent, and preferably radiopaque.

3. Composite according to any one of the preceding claims, which is in the form of an unidirectional composite tape, or in the form of a strand, rod, pellet or granule, in particular in the form of an unidirectional tape.

4. Composite according to any one of the preceding claims, wherein the inherent viscosity of the matrix polymer in the composite is between 1.5 and 4.0 dL/g, preferably between 1.8 and 3.0 dL/g, more preferably between 2.0 and 3.0 dL/g.

5. Medical device, such as an implant or scaffold, comprising a composite according to any one of the preceding claims.

6. Method for manufacturing a solid polymer composite comprising a plurality of biocompatible and resorbable glass fibers which are embedded in a biocompatible and resorbable matrix polymer, in particular a composite in accordance with any one of claims 1-5, comprising the steps of:
a) providing a plurality of biocompatible and resorbable glass fibers compatible with the matrix polymer,
b) providing a mixture comprising the matrix polymer in a solvent,
c) applying the mixture onto the plurality of glass fibers, and
d) removing the solvent from the mixture that has been applied onto the plurality of glass fibers using an anti-solvent to obtain the solid polymer composite material.

7. Method according to claim 6, wherein the solid polymer composite is shaped, preferably into a tape, strand, (cannulated) rod, tube, pellet, granule, or filament.

8. Method according to any one of claims 6 or 7, wherein the solvent is allowed to partially evaporate after it has been applied onto the glass fibers.

9. Method according to any one of claims 6-8 , wherein the matrix polymer is a thermoplastic polyester, in particular a polymer selected from the group consisting of polylactides (PLA), poly-L-lactide (PLLA), poly-DL-lactide (PDLLA), polyglycolide (PGA), poly(ε-caprolactone) (PCL), copolymers of glycolide, glycolide/trimethylene carbonate copolymers (PGA/TMC), lactide/tetramethylglycolide copolymers, lactide/trimethylene carbonate copolymers, lactide/d-valerolactone copolymers, lactide/ε-caprolactone copolymers, L-lactide/DL-lactide copolymers (PLDLA), glycolide/L-lactide copolymers (PLGA), polylactide-co-glycolide, lactide/glycolide/trimethylene carbonate terpolymers, lactide/glycolide/ε-caprolactone terpolymers, PLA/polyethylene oxide copolymers, poly(ε-caprolactone-DL-lactide) copolymers and combinations thereof may be preferred, in particular polymers selected from the group of polylactide, poly(lactide-co-glycolide), poly(lactide-co- ε-caprolactone), polyglycolide (PGA), and poly(ε-caprolactone) (PCL), and combinations thereof.

10. Method according to any one of claims 6-9, wherein the method comprises
- providing a second mixture comprising a second matrix polymer and a second solvent,
- applying the second mixture onto the plurality of glass fibers onto which matrix polymer has been applied, and
- optionally partially removing the second solvent from the second mixture that has been applied onto the plurality of glass fibers onto which the matrix polymer has been applied using a second anti-solvent to obtain the solid polymer composite material.

11. Method according to any one of claims 6-10, wherein the polymer concentration in the second mixture is higher than in the mixture that was previously applied.

12. Method according to any one of claims 6-11, wherein the method comprises removing residual solvent and/or anti-solvent by evaporation.

13. Method according to any one of claims 6-12, wherein the ratio between the inherent viscosity of the matrix polymer in the solid composite and inherent viscosity of the virgin matrix polymer is between 0.70 and 1.30, preferably between 0.90 and 1.10, more preferably between 0.95 and 1.05.

14. Method according to any one of claims 6-13, wherein the difference in Yellowness Index between the virgin matrix polymer and the composite obtained is less than 20, more preferably less than 10 as measured according to ASTM E313.

15. Method according to any one of claims 6-14, further comprising the step of embedding an additive, preferably an active pharmaceutical ingredient (API) or a mineral phase, in the composite.
